# FASCICULE DE BREVET EUROPEEN

(11) **EP 0 690 852 B1**
(45) Date de publication et mention de la délivrance du brevet: **26.02.1997**
(21) Numéro de dépôt: 94910438.4
(22) Date de dépôt: 18.03.1994
(51) Int. Cl.: C07D 305/14, A61K 31/335

(54) **PROCEDE DE PURIFICATION DES TAXOIDES**
VERFAHREN ZU REINIGUNG VON TAXOL DERIVATE
METHOD FOR PURIFYING TAXOIDS

(30) Priorité: 22.03.1993 FR 9303251
(43) Date de publication de la demande: 10.01.1996
(73) Titulaire: RHONE-POULENC RORER S.A., 92160 Antony (FR)
(72) Inventeur: DURAND, André, F-91700 Sainte-Geneviève-des-Bois (FR); GERBAUD, Alain, F-91200 Athis-Mons (FR); MARGRAFF, Rodolphe, F-91170 Viry-Châtillon (FR)
(74) Mandataire: Pilard, Jacques
(86) Numéro de dépôt international: FR9400300
(87) Numéro de publication internationale: WO9421622

(56) Documents cités:
- JOURNAL OF LIQUID CHROMATOGRAPHY vol. 15, no. 4 , 1992 pages 697 - 706 R. VANHAELEN ET AL. 'HIGH SPEED COUNTERCURRENT CHROMATOGRAPHY SEPARATION OF TAXOL AND RELATED DI TERPENOIDS FROM TAXUS BACCATA.'
- JOURNAL OF CHROMATOGRAPHY vol. 587 , 1991 , AMSTERDAM NL pages 300 - 305 S. HARVEY ET AL. 'SEPARATION OF TAXOL FROM RELATED TAXANES IN TAXUS BREVIFOLA EXTRACTS BY ISOCRATIC ELUTION REVERSED-PHASE HIGH-PERFORMANCE LIQUID CHROMATOGRAPHY.'

## Description

La présente invention concerne un procédé de purification de taxoïdes par chromatographie de partage centrifuge.

Plus particulièrement, la présente invention concerne un procédé de purification du Taxotère et de la désacétyl-10 baccatine III.

La désacétyl-10 baccatine III extraite des feuilles d'ifs est utile dans la préparation du Taxotère dans les conditions décrites par exemple dans les brevets européens EP 0 253 738 ou EP 0 336 841 ou dans la demande internationale PCT WO 92/09589.

La désacétyl-10 baccatine III, obtenue par extraction des feuilles d'ifs, contient, en fonction des espèces dont elle est issue, des impuretés qui sont essentiellement des produits appartenant à la famille des taxoïdes tels que, par exemple, le benzoyloxy-2α acétoxy-4 époxy-5β,20 pentahydroxy-1β,7β,10β,13α,19 oxo-9 taxène-11, le benzoyloxy-2α acétoxy-4 époxy-5β,20 tétrahydroxy-1β,7α,10β, 13α taxène-11 ou des taxines.

Le Taxotère qui est obtenu par hémi-synthèse à partir de la désacétyl-10 baccatine III contient comme impuretés essentielles les produits d'estérification des impuretés contenues dans la désacétyl-10 baccatine III ainsi que d'autres impuretés provenant, par exemple, de l'épimérisation du carbone en 2' de la chaîne latérale.

Dans Journal of liquid Chromatography, 15(4), 697-706 (1992), est décrite la séparation d'un mélange de taxol et de céphalomannine à partir d'extraits de t. Baccata en uutilisant une technique de chromatographie à contre-courant à grande vitesse. Cependant, le taxol n'est obtenu qu'avec une pureté de 97 %.

La désacétyl-10 baccatine III et le Taxotère lui-même peuvent être généralement purifiés par des méthodes de chromatographie liquide sur colonne et plus particulièrement par chromatographie liquide à haute performance (HPLC) sur colonne de silice, tele que décrite, par exemple, dans J. of Chromatography, 587, 300-305 (1991). Cependant, si ces méthodes sont particulièrement adaptées à la purification de quelques dizaines de grammes, leur extrapolation industrielle se heurte à des contraintes, telles que la quantité de solvants consommés, la manipulation et la destruction du support (silice) contaminé par des résidus toxiques, qui deviennent primordiales.

Dans le cas des taxoïdes qui constituent des produits dont la toxicité est souvent très élevée et dont la fragilité est grande, il est particulièrement important de disposer de méthodes de purification qui ont une bonne productivité, qui ne nécessitent pas de support solide coûteux à l'achat, à l'emploi et à la destruction, qui nécessitent la mise en oeuvre de faibles quantités de solvants et qui soient facilement automatisables pour permettre une production continue.

Il a maintenant été trouvé, et c'est ce qui fait l'objet de la présente invention, que la purification des taxoïdes, et plus particulièrement la purification du Taxotère et de la désacétyl-10 baccatine III, peut être réalisée par la mise en oeuvre de la chromatographie de partage centrifuge (CPC) ou chromatographie à contre-courant à grande vitesse dont le principe est décrit, par exemple, par A. Foucault et P. Rosset, Analusis, 16(3), 157-167 (1988).

La chromatographie de partage centrifuge permet d'effectuer le partage des constituants du mélange à séparer entre deux phases liquides non miscibles par des mises en équilibre successives réalisées de façon logique et automatique.

Cette méthode est caractérisée par un mécanisme de partage très efficace, une forte rétention de la phase stationnaire et une grande vitesse de la phase mobile conduisant à d'excellentes séparations en quelques heures.

La chromatographie de partage centrifuge nécessite la mise en oeuvre de deux ou plusieurs solvants fournissant 2 phases partiellement miscibles. Bien qu'il existe un nombre illimité de solvants présentant cette caractéristique, il est particulièrement important industriellement de mettre en oeuvre des solvants industriels non chlorés et non toxiques. Parmi les solvants qui peuvent être utilisés industriellement à moindres risques peuvent être cités les alcools tels que le méthanol, les éthers tels que le méthyl t.butyléther, les esters tels que l'acétate d'éthyle, les cétones telles que la méthylisobutylcétone et les hydrocarbures aliphatiques tels que l'heptane ou l'isooctane.

Pour la réalisation d'une purification efficace, il est nécessaire de choisir un mélange de solvants dont la séparation rapide et complète conduit à deux phases pour lesquelles le coefficient de partage est compris entre 0,1 et 10 et de préférence entre 0,5 et 5 et plus particulièrement encore au voisinage de 1.

Un produit pour lequel le coefficient de partage (K) est, par exemple, égal à 10 est 10 fois plus soluble dans la phase supérieure que dans la phase inférieure. En conséquence, il sera élué très rapidement si la phase supérieure est mobile et au bout d'un temps très long, avec une mauvaise résolution, si c'est la phase inférieure qui est mobile. En revanche lorsque K est voisin de 1, la solubilité sera pratiquement identique dans les deux phases et les deux modes d'élution pourront être utilisés, et éventuellement combinés, pour obtenir une résolution maximale.

Dans le cas particulier de la désacétyl-10 baccatine III, conviennent particulièrement bien des mélanges d'un hydrocarbure aliphatique, d'un ester, d'un alcool et d'eau tel que le mélange heptane-acétate d'éthyle-méthanol-eau (1-2-1-2 en volumes) et plus particulièrement des mélanges de cétones aliphatiques et d'eau tels que le mélange méthylisobutylcétone-acétone-eau (2-3-2 en volumes).

De plus, il est nécessaire que la rétention en phase stationnaire, qui est la fraction du volume total de l'appareil occupé par la phase stationnaire à l'équilibre, le débit de la phase mobile étant fixe, soit la plus élevée possible et au moins égale à 50%.

De plus, afin d'améliorer la productivité, il est particulièrement intéressant de pouvoir travailler à un débit élevé, qui est limité par la pression maximale imposée par le rotor.

Dans le cas particulier du Taxotère, il est avantageux d'utiliser un mélange d'un hydrocarbure aliphatique, d'un ester, d'un alcool et d'eau tel qu'un système heptane-acétate d'éthyle-méthanol-eau (2-4-3-2 en volumes) qui a une rétention de 80 % et un coefficient de partage voisin de 0,5 donnant ainsi un pic plus étroit qui est élué rapidement conduisant ainsi à une amélioration très nette de la productivité.

La chromatographie de partage centrifuge peut être mise en oeuvre dans tout appareil commercialisé adapté à cet effet tel que ceux qui ont été mis au point par Yoichiro Ito [CRC Crit. Rev. Anal. Chem., 17, 65 (1986)] ou commercialisés par SANKI Engineering Limited à Kyoto (Japon).

La présente invention concerne également le Taxotère et la désacétyl-10 baccatine III purifiés lorsqu'ils sont obtenus par la mise en oeuvre du procédé selon l'invention.

Les exemples suivants montrent comment l'invention peut être mise en pratique.

### EXEMPLE 1 - Purification du Taxotère

Le Taxotère (ou t.butoxycarbonylamino-3 phényl-3 hydroxy-2 propionate-(2R,3S) d'acétoxy-4 benzoyloxy-2a époxy-5β,20 trihydroxy-1α,7β,10β oxo-9 taxène-11 yle-13α) est obtenu par traitement par le zinc en présence d'acide acétique du t.butoxycarbonylamino-3 phényl-3 hydroxy-2 propionate-(2R,3S) d'acétoxy-4 benzoyloxy-2α époxy-5β,20 hydroxy-1β bis-(trichloro-2,2,2 éthoxycarbonyloxy)-7β,10β oxo-9 taxène-11 yle-13α) dans les conditions décrites dans le brevet européen EP 0 336 841.

On dissout 10 g de t.butoxycarbonylamino-3 phényl-3 hydroxy-2 propionate-(2R,3S) d'acétoxy-4 benzoyloxy-2α époxy-5β,20 hydroxy-1β bis-(trichloro-2,2,2 éthoxycarbonyloxy)-7β,10β oxo-9 taxène-11 yle-13α dans 150 cm3 de méthyl t.butyléther puis ajoute 10 g de zinc. Après évaporation à sec, on obtient une poudre grise qui est placée dans un réacteur.

On agite puis on ajoute en 5 minutes un mélange de 6 cm3 d'acide acétique et de 50 cm3 d'acétonitrile. On chauffe, extérieurement avec un bain d'eau à 45°C, pendant 1 heure. Après refroidissement dans un bain de glace, on introduit 850 cm3 de méthyl t.butyléther sous forte agitation puis filtre les sels de zinc et le zinc en excès sur verre fritté de porosité 4.

Le filtrat est concentré à sec. On obtient ainsi une meringue (12 g) que l'on reprend immédiatement dans 20 cm3 d'acétate d'éthyle, 15 cm3 de méthanol, 10 cm3 d'heptane et 10 cm3 d'eau.

La chromatographie de partage centrifuge est effectuée sur un appareil SANKI LLI-07. La centrifugeuse est alimentée par 2 pompes à piston équipées de vannes-soupapes limitant la pression à 55 bars. Une pompe sert à véhiculer l'une ou l'autre des deux phases grâce à une vanne de sélection ; l'autre pompe est réservée pour l'injection.

Après remplissage de l'appareil en phase stationnaire sous faible rotation (200 tours/minute) et fort débit (110 cm3/minute), l'injection est effectuée immédiatement.

On injecte les deux phases en commençant par la phase aqueuse et élue, à un débit de 60 cm3/minute, avec la phase aqueuse du système hexane-acétate d'éthyle-méthanol-eau (2-4-3-2 en volumes). On recueille 1,9 litre de phase stationnaire, soit une rétention de 71 %, puis recueille des fractions de 15 cm3. Les fractions 72 à 110 sont rassemblées et concentrées jusqu'à un volume de 140 cm3. Le précipité formé est séparé par filtration. On obtient ainsi, avec un rendement de 80,5 %, 5,2 g de taxotère trihydraté titrant 99,7 % en normalisation interne.

### EXEMPLE 2 - Purification du Taxotère

En opérant comme dans l'exemple 1 mais à partir de 82 g de t.butoxycarbonylamino-3 phényl-3 hydroxy-2 propionate-(2R,3S) d'acétoxy-4 benzoyloxy-2α époxy-5β,20 hydroxy-1β bis-(trichloro-2,2,2 éthoxycarbonyloxy)-7β,10β oxo-9 taxène-11 yle-13α, de 82 g de zinc et de 150 cm3 d'un mélange de 49,2 cm3 d'acide acétique et de 328 cm3 d'acétonitrile, on obtient 115 g de produit brut renfermant, en théorie, 49,69 g de Taxotère ou 53 g de Taxotère trihydraté.

On reprend le produit brut par 450 cm3 de méthyl t.butyléther puis lave par 3 fois avec au total 450 cm3 d'eau contenant 15 g de chlorure de sodium. Les phases aqueuses réunies sont extraites par 2 fois 200 cm3 de méthyl t.butyléther. Les trois extraits éthérés sont réunis et concentrés à sec. On obtient ainsi 72 g d'une meringue qui est reprise par 120 cm3 de méthanol.

A la solution jaune pâle obtenue, on ajoute, dans l'ordre, 160 cm3 d'acétate d'éthyle, 80 cm3 d'eau et 80 cm3 d'heptane.

On obtient ainsi 2 phases liquides limpides d'un volume total de 460 cm3 et dont la couche supérieure ne représente qu'environ 1/4 du volume total au lieu de 35 % en l'absence de Taxotère.

L'appareil de chromatographie de partage centrifuge est rempli avec la phase organique à un débit de 110 cm3/minute avec une vitesse de rotation de 200 tours/minute. L'appareil étant plein, la vitesse de rotation est portée à 800 tours/minute. On injecte la phase aqueuse, puis après injection de la phase organique, on rince avec 200 cm3 de phase organique puis élue, à un débit de 60 cm3/minute, avec la phase aqueuse.

On recueille 2,5 litres de phase stationnaire en excès, soit une rétention de 67,7 %.

Après passage de 200 cm3 de phase mobile aqueuse, on recueille 360 fractions de 12 à 13 cm3. Les fractions 25 à 110, concentrées jusqu'à un volume de 200 cm3, fournissent, avec un rendement de 87,7 %, un précipité de 46,5 g de Taxotère trihydraté dont la pureté est de 99,1 %.

### EXEMPLE 3 - Purification de la désacétyl-10 baccatine III

On effectue la chromatographie de partage centrifuge sur un chromatographe SANKI HPCPC série 1000 d'un volume total de 245 cm3.

Le rotor en rotation à 100 tours/minute est rempli en mode descendant par pompages simultanés de la phase organique au débit de 6 cm3/minute et de la phase aqueuse au débit de 3 cm3/minute du mélange méthylisobutylcétone-acétone-eau (2-3-2 en volumes).

La vitesse de rotation est ensuite portée à 1200 tours/minute puis on injecte 1,5 g de désacétyl-10 baccatine III brute cristallisée contenant 83,3 % de désacétyl-10 baccatine III et 3,4 % de désacétyl-10 hydroxy-19 baccatine III.

On élue ensuite en mode descendant avec la phase aqueuse au débit de 3 cm3/minute en recueillant des fractions de 6 cm3 toutes les 2 minutes.

La désacétyl-10 hydroxy-19 baccatine III est contenue dans les fractions 39 à 52.

A la 61ème fraction, le sens d'élution est inversé en utilisant alors la phase organique comme phase mobile en mode ascendant au débit de 5 cm3/minute et en recueillant des fractions de 10 cm3 toutes les 2 minutes.

Les fractions 70 à 84, qui contiennent la désacétyl-10 baccatine III, sont concentrées sous pression réduite. Après recristallisation dans 15 cm3 d'acétonitrile, on obtient 1,25 g de désacétyl-10 baccatine III solvatée par l'acétonitrile (1 mole/ mole) sous forme de cristaux blancs dont la pureté, déterminée par HPLC en normalisation interne, est de 98 %.

Le rendement, corrigé, est de 91,2 %.

## Revendications

1. Procédé de purification de la désacétyl-10 baccatine III et du Taxotère par chromatographie de partage centrifuge entre deux phases partiellement miscibles constituées d'eau et de solvants non chlorés et non toxiques choisis parmi les alcools, les éthers, les esters, les cétones et les hydrocarbures aliphatiques, caractérisé en ce que, pour purifier la désacétyl-10 baccatine III, on utilise un mélange d'un hydrocarbure aliphatique, d'un ester, d'un alcool et d'eau dont le coefficient de partage entre les deux phases est compris entre 0,1 et 10 ou un mélange de cétones aliphatiques et d'eau dont le coefficient de partage entre les deux phases est compris entre 0,1 et 10 et, pour purifier le Taxotere, on utilise un mélange d'un hydrocarbure aliphatique, d'un ester, d'un alcool et d'eau dont le coefficient de partage entre les deux phases est compris entre 0,1 et 10.

2. Procédé selon la revendication caractérisé en ce que le coefficient de partage entre les deux phases est compris entre 0,5 et 5.

3. Procédé selon la revendication 1 caractérisé en ce que le coefficient de partage entre les deux phases est voisin de 1

4. Procédé selon lune des revendications 1 à 3 revendication 1 caractérisé en ce que, pour séparer la désacétyl-10 baccatine III, on utilise un mélange d'heptane, d'acétate d'éthyle, de méthanol et d'eau.

5. Procédé selon la revendication 4 caractérisé en ce que l'on utilise un mélange heptane-acétate d'éthyle-méthanol-eau (1-2-1-2 en volumes).

6. Procédé selon l'une des revendications 1 à 3 caractérisé en ce que l'on utilise un mélange de méthylisobutylcétone, d'acétone et d'eau.

7. Procédé selon la revendication 6 caractérisé en ce que l'on utilise un mélange méthylisobutylcétone-acétone-eau (2-3-2 en volumes).

8. Procédé selon l'une des revendications 1 à 3 caractérisé en ce que, pour purifier le Taxotère, on utilise un mélange d'heptane, d'acétate d'éthyle, de méthanol et d'eau.

9. Procédé selon la revendication 8 caractérisé en ce que l'on utilise un mélange heptane-acétate d'éthyle-méthanol-eau (2-4-3-2 en volumes).

10. Le trihydrate du t.butoxycarbonylamino-3 phényl-3 hydroxy-2 propionate-(2R,3S) d'acétoxy-4 benzoyloxy-2α époxy-5β,20 trihydroxy-1β,7β,10β oxo-9 taxène-11 yle-13α ou trihydrate du Taxotère.

## Patentansprüche

1. Verfahren zur Reinigung von 10-Desacetyl-Baccatin III und Taxoter durch Zentrifugal-Verteilungschromatographie zwischen zwei teilweise mischbaren Phasen, bestehend aus Wasser und nicht chlorierten und nicht toxischen Lösungsmitteln, ausgewählt unter den Alkoholen, den Ethern, den Estern, den Ketonen und den aliphatischen Kohlenwasserstoffen, dadurch gekennzeichnet, daß man für die Reinigung von 10-Desacetyl-Baccatin III eine Mischung von einem aliphatischen Kohlenwasserstoff, einem Ester, einem Alkohol und Wasser verwendet, deren Verteilungskoeffizient zwischen den zwei Phasen von 0,1 bis 10 beträgt, oder eine Mischung von aliphatischen Ketonen und Wasser verwendet, deren Verteilungskoeffizient zwischen den zwei Phasen von 0,1 bis 10 beträgt, und für die Reinigung von Taxoter eine Mischung von einem aliphatischen Kohlenwasserstoff, einem Ester, einem Alkohol und Wasser verwendet, deren Verteilungskoeffizient zwischen den zwei Phasen von 0,1 bis 10 beträgt.

2. Verfahren nach Anspruch 1, dadurch gekennzeichnet, daß der Verteilungskoeffizient zwischen den zwei Phasen zwischen 0,5 und 5 liegt.

3. Verfahren nach Anspruch 1, dadurch gekennzeichnet, daß der Verteilungskoeffizient zwischen den zwei Phasen etwa 1 beträgt.

4. Verfahren nach einem der Ansprüche 1 bis 3, dadurch gekennzeichnet, daß man für die Abtrennung von 10-Desacetyl-Baccatin III eine Mischung von Heptan, Ethylacetat, Methanol und Wasser verwendet.

5. Verfahren nach Anspruch 4, dadurch gekennzeichnet, daß man eine Mischung von Heptan/Ethylacetat/Methanol/Wasser (1/2/1/2 in Volumen) verwendet.

6. Verfahren nach einem der Ansprüche 1 bis 3, dadurch gekennzeichnet, daß man eine Mischung von Methylisobutylketon, Aceton und Wasser verwendet.

7. Verfahren nach Anspruch 6, dadurch gekennzeichnet, daß man eine Mischung von Methylisobutylketon/Aceton/Wasser (2/3/2 in Volumen) verwendet.

8. Verfahren nach einem der Ansprüche 1 bis 3, dadurch gekennzeichnet, daß man für die Reinigung von Taxoter eine Mischung von Heptan, Ethylacetat, Methanol und Wasser verwendet.

9. Verfahren nach Anspruch 8, dadurch gekennzeichnet, daß man eine Mischung von Heptan/Ethylacetat/Methanol/Wasser (2/4/3/2 in Volumen) verwendet.

10. Das Trihydrat von 3-Tert.-Butoxycarbonylamino-3-phenyl-2-hydroxy-propionat-(2R,3S) von 4-Acetoxy-2a-benzoyloxy-5β,20-epoxy-1ß,7ß,10ß-trihydroxy-9-oxo-11-taxen-13a-yl oder Trihydrat von Taxoter.

## Claims

1. Process for the purification of 10-de-acetylbaccatin III and of Taxotere by centrifugal partition chromatography between two partially miscible phases consisting of water and non-chlorinated and non-toxic solvents chosen from alcohols, ethers, esters, ketones and aliphatic hydrocarbons, characterized in that, to purify 10-deacetylbaccatin III, a mixture of an aliphatic hydrocarbon, an ester, an alcohol and water whose partition coefficient between the two phases is between 0.1 and 10, or a mixture of aliphatic ketones and water whose partition coefficient between the two phases is between 0.1 and 10, is used, and, to purify Taxotere, a mixture of an aliphatic hydrocarbon, an ester, an alcohol and water whose partition coefficient between the two phases is between 0.1 and 10 is used.

2. Process according to claim 1, characterized in that the partition coefficient between the two phases is between 0.5 and 5.

3. Process according to claim 1, characterized in that the partition coefficient between the two phases is in the region of 1.

4. Process according to one of claims 1 to 3, characterized in that, to separate 10-deacetylbaccatin III, a mixture of heptane, ethyl acetate, methanol and water is used.

5. Process according to claim 4, characterized in that a heptane/ethyl acetate/methanol/water (1:2:1:2 by volume) mixture is used.

6. Process according to one of claims 1 to 3, characterized in that a mixture of methyl isobutyl ketone, acetone and water is used.

7. Process according to claim 6, characterized in that a methyl isobutyl ketone/acetone/water (2:3:2 by volume) mixture is used.

8. Process according to one of claims 1 to 3, characterized in that, to purify Taxotere, a mixture of heptane, ethyl acetate, methanol and water is used.

9. Process according to claim 8, characterized in that a heptane/ethyl acetate/methanol/water (2:4:3:2 by volume) mixture is used.

10. 4-Acetoxy-2α-benzoyloxy-5β,20-epoxy-1β,7β,10β-trihydroxy-9-oxo-11-taxen-13α-yl (2R,3S)-3-t-butoxycarbonylamino-3-phenyl-2-hydroxypropionate trihydrate or Taxotere trihydrate.
